# EUROPEAN PATENT APPLICATION

(11) **EP 3 583 898 A1**
(43) Date of publication of application: **25.12.2019**
(21) Application number: 18178237.6
(22) Date of filing: 18.06.2018
(51) Int. Cl.: A61B 5/08, A61B 5/11

(54) **REDUCING INHALATION OF POLLUTANTS WHEN TRAVELLING**

(71) Applicant: Koninklijke Philips N.V., 5656 AE Eindhoven (NL)
(72) Inventor: WRIGHT, Christopher John, 5656 AE Eindhoven (NL); LAWRENSON, Matthew John, 5656 AE Eindhoven (NL); BUIL, Vincentius Paulus, 5656 AE Eindhoven (NL); GEURTS, Lucas Jacobus Franciscus, 5656 AE Eindhoven (NL)
(74) Representative: de Haan, Poul Erik

(57) **Abstract**

A method and system for determining a recommended level of exertion of an individual. A pollution level is identified and used to recommend a level of exertion that takes into account an amount of pollution inhaled by the individual.

## Description

### FIELD OF THE INVENTION

The present invention relates to the field of methods and systems for reducing exposure of an individual to pollution, and in particular to the inhalation of pollution.

### BACKGROUND OF THE INVENTION

Air pollution or airborne allergens can have significant short- and long-term effects on individuals who exercise or travel outdoors. There is therefore a desire to reduce or minimize the exposure of an individual to pollution/allergens when exercising or travelling.

One existing method of reducing exposure to air pollution includes the usage of pollution masks to reduce an inhaled pollution dose. These masks can vary widely in filter strategy, effectiveness and comfort. For example, the popular 3M N95 mask filters particulates but not other pollutants such as ozone or volatile organic compounds (VOCs). The efficiency of fibrous filters, which make up most mask filters, varies with flow rate and filtered particle size. Due to this, as well as through increased leakage around the sides of a mask, pollution masks become less effective at filtering particles during heavy exercise due to increased breathing rate and depth. Moreover, pollution masks can significantly affect individual comfort through increased temperature, humidity and breathing resistance, which is a particular problem during exercise.

Another known method of reducing exposure to air pollution is to use a routing program that spatially resolves pollution data to construct a "healthiest route" for an individual that minimizes the inhaled pollution during an active journey. However, the reduction varies based on the range of route options available, which may be limited.

### SUMMARY OF THE INVENTION

The invention is defined by the claims.

According to examples in accordance with an aspect of the invention, there is provided a system for determining a recommended exertion rate of an individual when travelling through an area, the system comprising: pollution sensing apparatus adapted to determine a pollution level of the area through which the individual is to travel; and a recommended exertion calculation unit adapted to calculate, based on the determined pollution level, a recommended exertion of the individual that takes into account the individual's resulting inhalation of pollution when travelling through the area.

Thus, the system determines a recommended level of exertion for an individual based on a level of pollution (e.g. amount of pollen, other particulates or harmful gases) in an area through which they are to travel. This is performed by determining or assessing a pollution level of an area through which the individual is to travel, and calculating a recommended exertion that takes into account the anticipated inhalation of pollutants/allergens of the individual when travelling through the area.

In this way, an individual can be recommended a level of exertion that results in a reduced amount of pollution/allergens being inhaled, compared to that of an individual travelling using a non-recommended level of exertion. Alternatively, an individual can be recommended one or more levels of exertion that results in less than a (medically acceptable) maximum level of pollutant being inhaled, e.g. whilst maximizing a speed of travel.

Thus, the present invention aims to provide a recommended exertion that takes account of an individual's inhalation of pollution, based on a pollution level of an area through which the individual is travelling or is to travel.

The travelling is a human-powered transportation (e.g. cycling, walking or running). In this way, increased exertion by an individual tends to increase a speed of travel.

It will also be understood that an increased level of exertion results in an individual breathing more heavily (i.e. faster and/or deeper), so that in any single breath the individual breathes more pollutants/allergens. However an increased level of exertion also results in the individual spending less time in an area (e.g. of heavy pollution), meaning that the individual is exposed to fewer pollutants/allergens over a period of time.

One embodiment of the invention therefore proposes to recommend a level of exertion that balances or takes into account at least these two factors. In other words, the present invention recognizes that a tradeoff exists between time spent travelling and depth/rate of breathing during said travel for minimizing/reducing the total pollution inhaled on a journey.

Other factors or characteristics may influence the recommended level of exertion, such as the individual's goals or capabilities, and will be described in more detail later. Thus, embodiments may aim to balance a plurality of factors to identify a recommended level of exertion.

In at least one embodiment, the recommended exertion calculation unit is further adapted to determine, using the determined pollution level, a relationship between amount of pollution inhaled by the individual and an exertion of the individual. This can be used to calculate a recommended level of exertion for the individual.

By way of example, the system may be adapted to calculate a breathing rate and/or depth of the individual and, using the pollution level, determine a relationship between an amount of pollution inhaled by the individual and an exertion of the individual. This can be used to recommend a level of exertion.

The amount of pollution inhaled by the individual may be, for example, an amount of pollution inhaled during a single breath or representative of an amount inhaled over a period of time, such as a pollution inhalation rate.

This provides a system able to take an individual's inhalation of pollution in order to recommend a level of exertion. Other methods will be apparent to the skilled person.

The system may further comprise exertion monitoring apparatus adapted to determine a current exertion of the individual, wherein the recommended exertion calculation unit is adapted to calculate a recommended adjustment to the individual's exertion based on the recommended exertion and the current exertion of the individual.

In this way, a current exertion of the individual may be monitored or estimated, and a recommended adjustment (for achieving the recommended level of exertion) can be suggested. This provides an individual with an intuitive mechanism to see how they are required to adjust their level of exertion. Such a system may therefore be reactionary to a current exertion of the individual.

Preferably, the exertion monitoring apparatus comprises a vital sign monitoring system adapted to monitor a vital sign of the individual.

A vital sign has been identified as being a particularly good indicator of a level of exertion of the individual. In particular, there is a correlation between level of vital sign and breathing/respiratory rate/volume of the individual, which significantly affects the individual's exposure to pollution.

The vital sign monitoring system may be adapted to monitor one or more of the individual's: respiratory rate, pulse rate, body temperature or blood pressure.

The exertion monitoring apparatus may instead monitor other characteristics of the individual or the terrain in the immediate vicinity of the individual, such as the individual's speed, acceleration or sweat rate, or the terrain's incline or surface roughness. These characteristics have also been identified as also being indicative of the current level of exertion of the individual.

In a preferable embodiment, the exertion monitoring apparatus comprises a respiratory rate monitor adapted to determine a current respiratory rate of the individual, representative of the current exertion of the individual.

Monitoring the individual's respiratory rate (i.e. breathing rate) has been identified as providing a highly accurate method of assessing the individual's level of exertion. In particular the respiratory rate can accurately indicate an amount of air (and thereby pollutants) that the individual is inhaling, and thereby being exposed to. By assessing the current exertion based on breathing rate, the amount of pollutants that are inhaled by the individual can be reduced.

The system may further comprise a sensory output system adapted to provide a sensory output indicative of the recommended exertion of the individual.

The individual can therefore be advised as to the recommended level of exertion. The sensory output system may provide one or more tactile, audio or visual outputs for recommending the level of exertion to the individual. Thus, the sensory output system may comprise one or more: tactile outputs (e.g. a vibrating member), audio outputs (e.g. a speaker) or visual outputs (e.g. a LED screen).

In an example where the individuals current exertion is monitored, the sensory output system may provide an indication of how the individual should adjust their exertion (e.g. increase exertion or decrease exertion) based on the recommended level of exertion and the individual's current exertion.

In some embodiments, the system further comprises an individual characteristic determining unit adapted to determine one or more characteristics of the individual, wherein the recommended exertion calculation unit is adapted to calculate the recommended exertion of the individual further based on the determined one or more characteristics of the individual.

Individual characteristics of the individual may therefore be used to modify or personalize the recommended exertion of the individual. It has been recognized that different individuals will have different capabilities or requirements that can affect their sensitivity to pollutants or their capabilities of providing a particular exertion.

This allows a recommended level of exertion to be tailored to an individual, providing a more personalized and therefore more appropriate recommended level of exertion. This increases a likelihood that the individual will follow the recommended level of exertion.

By way of example, individual characteristics may include one or more of an individual's: fitness level; age; gender; size; height; weight; pollutant/allergen sensitivity; capability of performing a certain exertion; mode or method of transportation (e.g. by bicycle, running, walking etc.); time taken for a breathing rate to change between changing an exertion level ("lag time") and so on.

A system may further comprise terrain determining apparatus adapted to determine one or more characteristics of the terrain in the area through which the individual is to travel, wherein the recommended exertion calculation unit is adapted to calculate the recommended exertion of the individual further based on the determined one or more characteristics of the terrain.

Characteristics of the terrain may affect the ability of an individual to adhere to a particular exertion level (e.g. due to difficulty of travelling) or may prevent the individual from travelling with a steady exertion level (e.g. due to obstacles along the terrain). Characteristics of the terrain may include one or more of: terrain incline, terrain roughness, terrain obstacles (e.g. road crossings, traffic, traffic flow controllers such as traffic lights etc.).

By calculating the recommended level of exertion based on characteristics of the terrain, a more appropriate and individual-specific recommended exertion can be provided. This increases a likelihood that the individual will follow the recommended level of exertion, and also allows characteristics of the route to be taken into account, thereby decreasing an amount of pollution inhaled by the individual.

The system may further comprise a route determining unit adapted to determine an intended route of the individual, wherein the recommended exertion calculation unit is adapted to calculate the recommended exertion of the individual further based on the intended route of the individual.

Thus, the recommended exertion may be based on characteristics of an intended route. By way of example, a plurality of recommended exertions may be generated for different points/sections along the intended route. This allows a more bespoke and appropriate recommended exertion to be calculated.

The pollution sensing apparatus is adapted to determine pollution levels along the intended route of the individual, wherein the recommended exertion calculation unit is adapted to calculate the recommended exertion of the individual further based on the determined pollution levels along the intended route of the individual.

In this way, a recommended exertion may take account of a changing pollution level along the route taken by the individual. This means that a recommended exertion may change along a route taken by the individual. This allows the recommended exertion level to more accurately reflect the pollution encountered by the individual.

The system may comprise an individual goal obtaining unit adapted to obtain one or more goals of the individual when travelling through the area, wherein the recommended exertion calculation unit is adapted to calculate the recommended exertion of the individual further based on the determined one or more goals of the individual.

The recommended exertion may therefore lie within a range defined by the individual's desired goals. In particular, an individual's goals may dictate a level of exertion that they wish to include or provide (e.g. minimum/maximum level of exertion). Thus, the recommended exertion calculation unit may be adapted to limit possible recommended exertions to only include levels of exertion based on such goals of the individual.

Possible individual goals include a minimum exertion level, a maximum exertion level or desire to perform a particular exertion level for a predetermined period of time or distance.

The recommended exertion of the individual may be a recommended speed of the individual. A recommended speed may be intuitively understood by the individual, and increases an individual's understanding of their recommended exertion, increasing a likelihood that they will adhere to the recommended level of exertion.

The system may comprise an individual assistance device adapted to reduce an exertion of the individual exertion when travelling, wherein the individual assistance device is adapted to vary a level of assistance based on the determined pollution level of the area through which the individual is to travel.

The system may further comprise enjoyment determining apparatus, adapted to predict an enjoyment of the individual travelling through the area. The recommended exertion calculation unit may calculate the recommended exertion further based on the predicted enjoyment of the individual.

In a similar manner to the individual goal obtaining unit, the enjoyment determining apparatus may define limits (e.g. upper or lower limits) for the recommended exertion of the individual based on a predicted enjoyment of the individual. That is, the recommended exertion may fall within a range defined by the enjoyment determining apparatus.

According to examples in accordance with an aspect of the invention, there is also provided a method for determining a recommended exertion rate of an individual when travelling through an area, the method comprising: determining a pollution level of the area through which the individual is to travel; and calculating, based on the determined pollution level, a recommended exertion of the individual that minimizes the individual's inhalation of pollution when travelling through the area.

The method may further comprise determining a current exertion of the individual; and calculating a recommended adjustment to the individual's exertion based on the recommended exertion and the current exertion of the individual.

There is also proposed a computer program comprising code means for implementing the previously described method when said program is run on a computer.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a system according to an embodiment of the invention;
Figure 2 illustrates a system according to another embodiment of the invention;
Figure 3 illustrates a system according to yet another embodiment of the invention;
Figure 4 illustrates an exemplary relationship between level of exertion and tidal volume of an individual;
Figure 5 illustrates an exemplary relationship between level of exertion and respiratory rate of an individual;
Figures 6 and 7 illustrate systems for providing an indication of a recommended exertion to an individual;
Figure 8 illustrates a system, comprising an individual assistance device, according to an embodiment; and
Figure 9 illustrates a method according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a method and system for determining a recommended level of exertion of an individual. A pollution level is identified and used to recommend a level of exertion that takes into account an amount of pollution inhaled by the individual.

Embodiments are at least partly based on the realization that an individual's level of exertion affects the amount of pollution that they inhale. Thus, by determining a pollution level in an area through which the individual will travel, a recommended exertion can be generated that takes account of the pollution that individual will inhale.

Illustrative embodiments may, for example, be employed in systems for recommending a level of exertion, such as fitness applications, or with automated assistance devices such as electrically assisting devices.

There is an underlying aim of the invention to determine and recommend an exertion level for an individual that takes account of pollution inhaled by the individual. For example, this may include determining an appropriate range of speeds or heart-rate range for the individual, which controls the exertion level for the individual.

The recommended exertion level preferably aims to ensure that an amount of pollution inhaled by the individual, e.g. in a single breath or accumulatively over the course of the journey, does not exceed an allowable or safe level. To do so, at least a pollution level(s) is used to determine the recommended exertion level. For example, a pollution level can be used to define a range of exertion levels of the individual that do not cause an inhalation of pollutants (over time or in a single breath) to exceed a recommended level.

However, other variables/factors may also be taken into account when determining the recommended exertion level. These other variables include goals or desires of the individual, characteristics or capabilities of the individual, characteristics of the terrain or a predicted enjoyment level of the individual.

In this way, it is an aim of an embodiment to minimize exposure to pollution, whilst also allowing an individual to achieve their goals and enjoy a journey. Examples of the effect of these variables will become apparent from the following described embodiments.

As used herein, the term "pollution" refers to the presence of any harmful, toxic or irritating substance in the air. Thus, the term "pollution" generally refers to air pollution, which may include pollen, exhaust fumes, NO₂, CO, CFCs, SO₂, dust, VOCs, particulates and so on.

The term "exertion" refers to a level of (perceived) physical effort provided by an individual when travelling through an area. Thus, the greater an exertion of an individual, the more physical effort the individual is providing to travel (e.g. in an effort to travel faster).

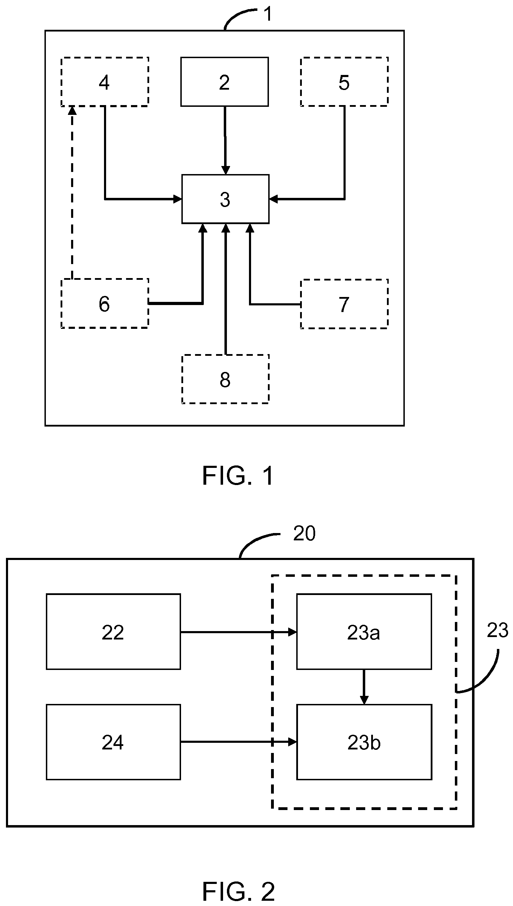

Figure 1 illustrates a framework for a system 1 according to an embodiment of the invention. The system 1 comprises pollution sensing apparatus 2 and a recommended exertion calculation unit 3. The system may also comprise one or more other optional units, illustrated in Figure 1 and later described.

The pollution sensing apparatus 2 is adapted to determine a pollution level of the area through which the individual is to travel. Thus, the pollution sensing apparatus 2 may sense pollution in the immediate vicinity of the individual or determine pollution in an upcoming area (e.g. along a route to be taken by the individual).

The pollution sensing apparatus 2 may, for example, comprise a pollution monitoring device or sensor adapted to directly detect the pollution in the vicinity of the individual. In other examples, the pollution sensing apparatus comprises location monitoring apparatus and a pollution mapping database (that maps pollution information to a location) to determine a level of pollution in an area through which the individual will travel (or is travelling). Other examples would be apparent to the skilled person.

The recommended exertion calculation unit 3 is adapted to calculate, based on the determined pollution level, a recommended exertion of the individual that takes into account the individual's resulting inhalation of pollution when travelling through the area. The recommended exertion of an individual may thereby provide an indicator to the individual of the level of physical effort that they should provide when travelling through the area.

By reducing a level of exertion or physical effort, an amount of pollution inhaled by the individual within a single breath is reduced. The recommended exertion calculation unit 3 may therefore be adapted to recommend a (maximum) exertion that keeps the individual's inhalation of pollution in a single breath below a predetermined value, such as a medically recommended maximum.

However, by reducing a physical effort, there is also a reduction in speed, meaning an individual may spend more time in a polluted area (i.e. take more breaths within a polluted area), and thereby inhale additional pollution over time.

Thus, in another example, the recommended exertion calculation unit 3 may calculate a recommended exertion that makes a balance between speed and breathing rate/depth to optimize the individual's overall inhalation of pollution (rather than on a breath by breath basis). Thus, the recommended exertion may aim to balance these two factors, and provide a recommended exertion that reduces or optimizes, or at least takes account of the individual's overall inhalation of pollution.

The recommended exertion of an individual may be one or more recommended characteristics of the individual that is affected by or otherwise represents their physical effort, such as a speed, heart rate, breathing rate and so on. Preferably, the recommended exertion calculation unit 3 calculates a recommended speed for the individual when traveling through the area to reduce the amount of pollution inhaled by the individual.

The system 1 may comprise an exertion monitoring apparatus 4 adapted to determine a current exertion of the individual. Thus, the exertion monitoring apparatus 4 assesses or estimates a current level of physical effort provided by the individual. This may be performed by monitoring characteristics of the individual or of the immediate terrain (which affects a level of physical effort required by the individual to traverse the terrain).

The recommended exertion calculation unit 3 may be adapted to calculate a recommended adjustment to the individual's exertion based on the recommended exertion and the current exertion of the individual.

Thus, the recommended exertion calculation unit 3 may receive as input a current exertion of the individual (from the exertion monitoring apparatus 4) and a pollution level of the area (from the pollution monitoring apparatus 2) through which the individual is to travel. The recommended exertion calculation unit may thereby recommend an adjustment to the individual's current exertion, based on the pollution level, to take into account the individual's inhalation of pollution.

The exertion monitoring apparatus 4 may employ various ways to assess, determine or estimate a current exertion of the individual.

For example, the exertion monitoring apparatus 4 may monitor one or more vital signs of the individual to assess a level of exertion provided by the individual, such as a heart rate, respiratory rate, blood pressure or body temperature. Monitoring vital signs provides a particularly accurate indication of an individual's level of exertion.

Other characteristics of the individual that could be monitored by the exertion monitoring apparatus 4, and which are also indicative of a current level of exertion, might include: speed/acceleration of the individual, stride frequency/turnover, amount of sweat, skin color, skin resistance/impedance, glucose level and so on. Such characteristics have also been shown to provide a good indicator of the individual's level of exertion.

In other examples, the exertion monitoring apparatus 4 monitors characteristics of the terrain that will affect a current exertion of the individual. For example, an individual will have a higher level of exertion on steep ground than flat ground. Thus, the exertion monitoring apparatus may estimate a current exertion by assessing characteristics of the terrain, such as incline, surface roughness, altitude and so on.

The recommended exertion calculation unit 3 may be adapted to consider other factors (e.g. the individual's capabilities, desires or goals) when calculating the recommended exertion. Hereafter described units/modules are used to allow the recommended exertion calculation unit to take account of such factors.

Each other factor may define a desired range for the recommended exertion calculation unit. For example, a first factor (e.g. individual's goals) may provide a lower limit for the recommended exertion, and a second factor (e.g. individual's capabilities) may provide an upper limit. The recommended exertion calculation unit may then be adapted to identify a recommended exertion that meets the requirements of the other factors, whilst also taking into account an individual's inhalation of pollution.

The system 1 may comprise an individual characteristic determining unit 5 adapted to determine one or more characteristics of the individual.

The recommended exertion calculation unit 3 may be adapted to calculate the recommended exertion of the individual further based on the determined one or more characteristics of the individual. Thus, the recommended exertion calculation unit 3 may take into account characteristics of the individual when determining a recommended exertion level for the individual.

In particular, the individual characteristic determining unit may be adapted to determine characteristics of the individual that may affect the level of exertion that they are able to produce. By way of example, the individual characteristic determining unit may determine a level of fitness of the individual. The higher the level of fitness, the greater an exertion that the individual is able to produce.

In an embodiment, the recommended exertion calculation unit is adapted to determine capabilities of the individual based on the individual characteristics, and only recommend a level of exertion within the capabilities of the individual. By way of example, an individual's capabilities may be represented by a percentage likelihood that an individual will adhere to a possible recommended exertion (e.g. speed), and the recommended exertion calculation unit may be adapted to only recommend an exertion for which the associated percentage likelihood is above a predetermined value (e.g. between 60-90%, such as around 75%).

Thus, the recommended exertion may be limited to the capabilities of the individual. This ensures that a recommended exertion is realistic, and does not exceed what the individual is able to perform.

Individual characteristics that may be determined by the individual characteristic determining unit include one or more of: tidal volume of the individual, breathing capacity of the individual, stride length, height, weight, age, gender, fitness level, maximum heart rate, resting heart rate, maximum speed, maximum exertion level, medical history (e.g. presence or absence of asthmas or COPD), past fitness information, allergen information, pollutant sensitivity, allergen sensitivity and so on. These characteristics may define a capabilities of the individual to adhere to an exertion, or may affect the individual's sensitivity to pollution (i.e. limit the amount of pollution that it is safe to inhale)

The system 1 may further comprise a terrain determining apparatus 6 adapted to determine one or more characteristics of the terrain in the area through which the individual is to travel.

The recommended exertion calculation unit 3 may accordingly be adapted to calculate the recommended exertion of the individual further based on the determined one or more characteristics of the terrain. Thus, the recommended exertion calculation unit 3 may take into account characteristics of the terrain when determining a recommended exertion level for the individual.

The characteristics of the terrain may affect a level of exertion supplied by an individual (i.e. affect the current level of exertion) or a maximum level of exertion that the individual is able to provide (e.g. speed). By considering terrain characteristics, a more accurate or realistic recommended exertion of the individual can be determined.

In some embodiments, the terrain determining apparatus 6 passes information about the characteristics of the terrain to the exertion monitoring apparatus 4, as the characteristics of the terrain may affect the exertion provided by the individual. By way of example, more physical effort is required to maintain a speed over rough terrain than over smooth terrain (i.e. an exertion is higher over rougher terrain for a same speed). In other words, the exertion monitoring apparatus 4 may receive information about terrain characteristics, and use this information when determining a current exertion of the individual (e.g. increasing a determined current exertion in response to determining that a terrain is steep).

The system 2 may comprise a route determining unit 7 adapted to determine an intended route of the individual. The recommended exertion calculation unit 3 may be accordingly adapted to calculate the recommended exertion of the individual further based on the intended route of the individual. Thus, the recommended exertion calculation unit 3 may take into account an intended route of the individual, or characteristics of the route, when determining a recommended exertion level for the individual.

The route taken by the individual (e.g. the length, intended locations and so on) will affect the level of exertion an individual is able to provide or the level of pollution along the route.

By way of example, if the individual intends to travel alongside a main road, they will experience more pollution than if the individual intends to travel along a country path. Thus, by taking into account the intended route of the individual, a recommended exertion calculation unit may be able to more accurately assess a dose of pollution that the individual will experience per inhalation.

By way of another example, if an individual intends to travel for a long period of time, they will be unable to maintain a high level of exertion (e.g. based on their fitness level). Thus, a recommended exertion level may take into account at least the length of an intended route in order to recommend a level of exertion to the individual, for example, to limit a maximum allowable recommended exertion based on a length of the route.

In other examples, the recommended exertion may differ along the intended route. Thus, the recommended exertion calculation unit 3 may be adapted to calculate a plurality of recommended exertions for different locations/segment/sections along the intended route.

The system 2 may comprise an individual goal obtaining unit 8 adapted to obtain one or more goals of the individual. The recommended exertion calculation unit is adapted to calculate the recommended exertion of the individual further based on the determined one or more goals of the individual. Goals or individual targets may therefore be used by the recommended exertion calculation unit to calculate a recommended level of exertion or to limit a range of allowable recommended levels of exertion.

The individual goal obtaining unit 8 may obtain the one or more goals, for example, from a user interface or from another device/unit that stores the individual's goals, such as a server adapted to store the individual's goals or a fitness/exercise plan (which may define the goals for the individual).

In one example, a goal of the individual may be to maintain a minimum level of exertion to adhere to a particular exercise regime. The recommended exertion calculation unit may take account of this goal of the individual, for example, to provide a minimum allowable recommended exertion to adhere to the goal.

By way of another example, a goal of the individual may be to provide a burst of speed or high level of exertion for a predetermined period of time. The recommended exertion calculation unit may be adapted to identify a best location to perform such a burst of speed to minimize their overall inhalation of pollution, for example, by indicating when it would be safe to provide a high level of exertion.

From the foregoing, it will therefore be appreciated that the recommended exertion calculation unit may be able to process variables from numerous sources to determine a recommended exertion of an individual that takes into account the individual's inhalation of pollution when travelling through the area.

In particular, different sources may set a range of allowable or desired levels of exertion from which the recommended exertion may be provide based on a determined pollution level. For example, an individual's capabilities (e.g. fitness level) may provide an upper limit for a recommended exertion level and an individual's goals/targets may provide a lower limit for a recommended exertion level.

Exemplary embodiments of devices described hereafter comprise a system that employs a number or selection of these sources to determine a recommended exertion of the individual.

Preferably, the system is incorporated into a portable device, such as a mobile phone, smartwatch or fitness tracker. The system may be integrated into a human-powered vehicle, such as a bicycle.

Figure 2 illustrates a device 20 employing a system according to an embodiment of the invention. The device 20 provides a recommended exertion that is reactionary to a current environment of the individual.

The device 20 comprises pollution sensing apparatus 22 adapted to sense pollution in the vicinity of the individual. The pollution sensing apparatus may comprise a pollution monitoring device (e.g. an ambient pollution sensor) or a location monitoring apparatus and a pollution mapping database adapted to map a current location of the individual to a known pollution level.

The pollution sensing apparatus 22 outputs pollution data indicative of a level of pollution in the immediate vicinity or current environment of the individual. The pollution data could indicate a type and/or intensity of pollution near the individual, such as a measure of the pollution level near the individual.

The device 22 also comprises an exertion monitoring apparatus 24 adapted to measure the individual's current exertion level. The exertion monitoring apparatus 24 may, for example, monitor an individual's heart rate, which is indicative of their current exertion.

The device 20 also comprises a recommended exertion calculation unit 23, here formed of two separate modules 23a, 23b. The recommended exertion calculation unit 23 is adapted to process the pollution data and the current exertion level of the individual in order to provide a recommended adjustment to the individual's exertion level.

A first module of the recommended exertion calculation unit 23 is an ideal exertion calculation module 23a. The ideal exertion calculation module is adapted to determine the ideal (i.e. recommended) exertion level for the individual based on the pollution data, i.e. for the current environment of the individual, that takes into account a inhalation of pollution. The ideal exertion calculation module 23a may comprise a look-up a table of target exertions (e.g. heart-rate zones) for different pollution types and/or intensities (i.e. different values of the pollution data).

A second module of the recommended exertion calculation unit 23 is an exertion adjustment system 23b adapted to recommend an adjustment to the exertion of the individual based on the ideal exertion for and the current exertion of the individual. By way of example, if the individual's current heart rate is above an ideal heart rate for their environment, the recommended exertion calculation unit may recommend the individual to reduce their heart rate or reduce their speed (which would in turn reduce their heart rate).

The recommended level of exertion does not to be the same measure used to identify a current level of exertion. By way of example, a current level of exertion may be represented by a current heart rate, and the recommended level of exertion may be expressed in terms of speed (e.g. to encourage the individual to speed up or slow down).

In this way, the device 20 provides a reactionary system for reducing or taking account of the inhalation of pollutants by recommending changes to an individual's exertion. This provides a highly accurate and low-cost method of calculating a recommended exertion level.

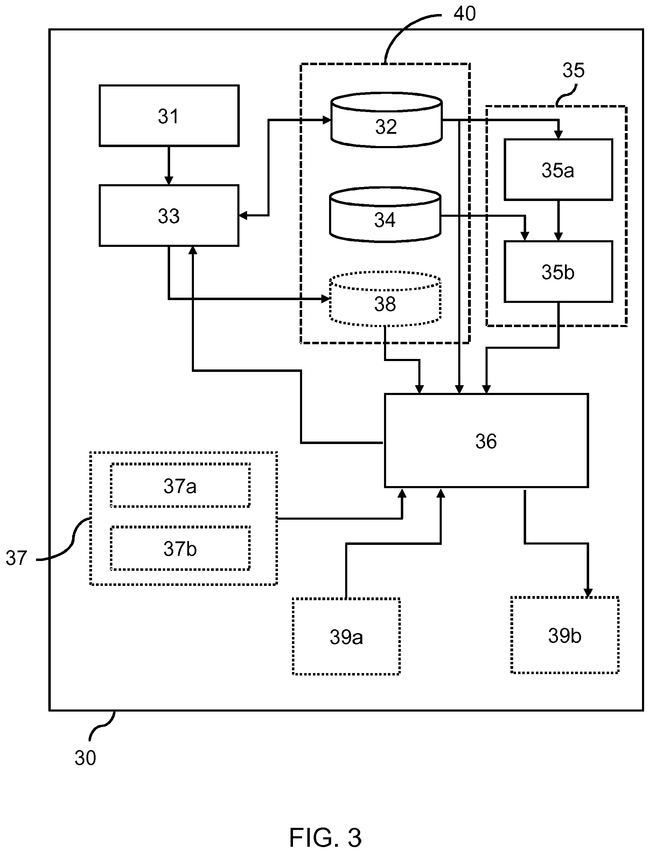

Figure 3 illustrates a device 30 employing a system according to another embodiment of the invention.

The device 30 of Figure 3 is adapted to calculate a recommended exertion for an individual travelling along a route. In particular, the system is adapted to associated different parts of the route with different recommended exertions for the individual.

The device 30 comprises individual tracking apparatus 31 adapted to track or monitor a location of the individual. The individual tracking apparatus 31 may use a satellite navigation apparatus to determine a location of the individual. In one example, the individual tracking apparatus tracks the coordinates of the individual on a 2D grid.

The device 30 also comprises a mapping database 32 containing pre-acquired mapping data about the environment around the individual. The mapping database may be stored in a memory system 40, and may be updated regularly, e.g. via an internet connection or the like. In other embodiments, the mapping database 32 (or part thereof) is stored externally to the device 30, e.g. on a server or a cloud computing network.

This mapping data may relate coordinates of a 2D grid to reference values referring to: the spatial distribution of pollution types and their intensities and the topology of the route, e.g. location of paths, slope intensities and so on. In other words, the mapping data may comprise information on at least pollution levels and terrain information of areas (such as location of paths/roads) around the individual. The mapping data may include information on speed interfering factors, such as timings of road crossings or estimated wait times.

The device 30 also comprises a user interface 33 adapted to display the mapping data and location of the individual, and to allow the individual to input a desired or intended route of travel. In some embodiments, routing software is used to define the intended route (e.g. by establishing a route from the individual's location to a selected location). The intended route may be pass to and stored by the mapping database 32. By way of example, the user interface 33 may comprise a touch-sensitive display (touch screen) or a combination of a display and individual input device (e.g. keyboard).

Thus, the individual tracking apparatus 31, mapping database 32 and user interface 33 together act as a route determining unit adapted to determine an intended route of the individual. Other embodiments for a route determining unit (e.g. a module for receiving an intended route from another device) will be apparent to the skilled person.

The device 30 also comprises an individual characteristic database 34 adapted to store characteristic data. The characteristic data comprises information about characteristics of the individual, such as fitness characteristics, or demographic information (e.g. age, gender, weight and so on). Fitness characteristics may be obtained by tracking the individual using wearables such as fitness trackers, smartwatches and smartphones. The individual characteristic database maybe stored in the memory system 40.

The device 30 also comprises a pollution dose calculator 35. The pollution dose calculator is adapted to determine, along different points of the intended route, a relationship between level of exertion and at least dose of pollution (i.e. the amount of inhaled pollution in a single breath).

The pollution dose calculator 35 comprises a route segmenter 35a. The route segmenter 35a divides the intended route into segments (i.e. areas or portions) that share similar pollution levels to provide a "segmented route". For example, a first segment of a route may be a section having approximately a first pollution level and a second segment of a route may be a section having approximately a second, different pollution level. Information on the pollution levels along the route can be obtained from the mapping database 32.

Optionally, the route segmenter 35a divides the intended route into areas/sections that share similar pollution levels and similar terrain characteristics (e.g. inclines, gradients or surface roughness/type). Thus, two different sections may have identical pollution levels, but different route characteristics and vice versa. Information on the characteristics of the route may also be obtained from the mapping database 32.

Thus, each segment of a segmented route is associated with a particular pollution level.

The pollution dose calculator 35 comprises a relationship determiner 35b. The relationship determiner 35b may determine, for each segment of the segmented route, a relationship between an exertion of the individual and an estimated amount of inhaled pollution.

In particular, the relationship determiner 35b may use characteristic data from the individual characteristic database 34 to estimate a breathed air volume for different levels of exertion. A breathed air volume is an amount of air or gas inhaled by the individual in a single breath, which differs based on exertion level. This determination may be performed, for example, by referencing a database correlating individual characteristics to an estimated breathed air volume or using a function linking the same.

Thus, the relationship determiner 35b determines a relationship between an exertion of the individual and an estimated amount of inhaled air/gas in a single breath.

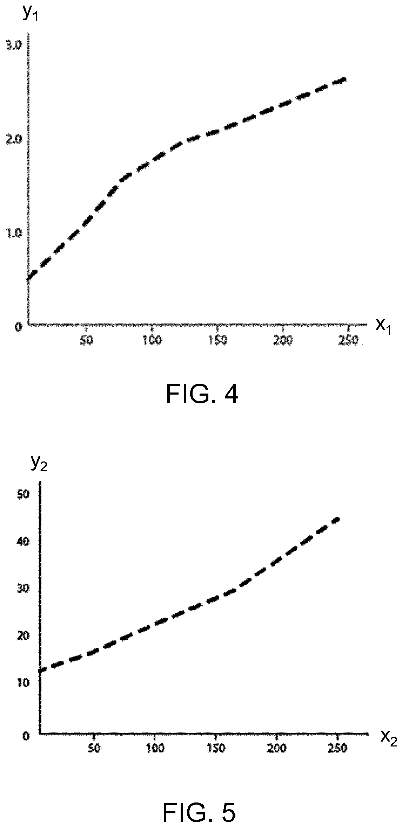

Figure 4 illustrates an exemplary (e.g. default) relationship between exertion of an individual and estimated tidal volume - i.e. amount of gas inhaled in a single breath. The exertion of the individual x₁ is here expressed in Watts (W), i.e. joules per second (Js⁻¹), and the tidal volume y₁ in liters (1). The illustrated relationship is representative of an average individual.

It will be appreciated that individual characteristics will affect the volume of air inhaled in a single breath. Some such individual characteristics include: age, gender, weight, fitness level, method of transportation, height, demographic and so on.

The relationship determiner 35b may then use this relationship together with information on the pollution level of each segment of the segmented route to estimate an amount of inhaled pollution in a single breath for different levels of exertion. An exertion level can therefore be associated with an estimated amount of inhaled pollution.

Thus, the relationship determiner may calculate a function or equation that receives, as input, at least an exertion level (e.g. speed or heart rate) of an individual and outputs an estimated amount of pollution inhaled in a single breath when travelling through a given segment and vice versa.

Thus, the relationship determiner 35b determines a relationship between an exertion of the individual and an estimated amount of inhaled pollution in a single breath. This relationship may be referred to as an estimated dose curve.

Referring back to Figure 3, the device 30 also comprises a recommended exertion calculation unit 36 adapted to calculate, based on the output of the relationship determiner, a recommended exertion of the individual for each segment, that takes accounts of the individual's estimated inhalation of pollution when travelling through that segment.

In preferable embodiments, the recommended exertion calculation unit is adapted to determine a recommended speed of the individual. Accordingly, the relationship determiner can be adapted to correlate an individual's speed with an estimated amount of pollution inhaled in a single breath.

In one example, the recommended exertion calculation unit 36 may identify a maximum allowable amount of inhaled pollution in a single breath (e.g. according to a medical standard or individual characteristics), and identify, for each segment, the highest exertion (e.g. speed) that is associated with an amount of inhaled pollution at or below this maximum allowable amount using the relationship established by the relationship determiner 35b. This highest exertion may be output as the recommended exertion.

In some embodiments, the relationship determiner 35b may determines a relationship between an individual's level of exertion and a breathing rate of the individual. This information may, for example, be stored in the individual characteristic database 34, or may be based on standard estimated values (e.g. estimate a breathing rate of around 35-40 breaths per minute for high-level exercise and around 12-20 for minimum level exercise). Alternatively, a determination may be performed by referencing a database correlating individual characteristics to an estimated breathing rate.

For the sake of understanding, Figure 5 illustrates an exemplary (e.g. default) relationship between individual's level of exertion x₂ and a breathing rate y₂ of the individual. The individual's level of exertion x₂ is here expressed in Watts (W), i.e. joules per second (Js⁻¹), and breathing rate in number of breaths per minute. The illustrated relationship is representative of an average individual.

The relationship determiner 35b can use the relationship between exertion and breathing rate and the relationship between exertion and volume of pollution inhaled in a single breath to calculate a relationship between volume of pollutant inhaled over time and level of exertion. Thus, the recommended exertion calculation unit 36 or the relationship determiner may determine a relationship, in each segment, between level of exertion (e.g. speed) and amount of pollution inhaled over time.

The recommended exertion calculation unit 36 may use this latter relationship to determine a recommended exertion for the individual in each segment.

By way of example, the recommended exertion calculation unit 36 may be adapted to identify a maximum allowable amount of inhaled pollution over a period of time (e.g. according to a medical standard) or rate of inhalation and determine a recommended exertion that results in this maximum allowable amount of inhaled pollution or rate.

In yet other examples, the recommended exertion calculation unit 36 maybe adapted to identify a level of exertion (e.g. speed) that minimizes the amount of pollution inhaled over time in each segment. Thus, the level of exertion may identify a level of exertion that balances a time spent in a segment with a rate and depth of breathing associated with that level of exertion.

In some further embodiments, the relationship determiner 35b may determine a relationship between an individual's level of exertion and a speed of the individual. This information may, for example, be stored in the individual characteristic database 34 (e.g. from data obtained from a fitness tacking device), be based on standard estimated values or be performed by referencing a database correlating individual characteristics to an estimated speed.

The recommended exertion calculation unit may use this relationship to determine a recommended exertion for the individual in each segment.

By way of example, the recommended exertion calculation unit may determine a maximum allowable amount of inhaled pollution over the course of a journey. If the relationship between exertion and speed, the relationship between exertion and pollution breathing rate and the length of the journey is known, it can be calculated how much pollution is inhaled by the individual completing the journey. Thus, a recommended exertion may be identified that balances these characteristics.

The minimum level of exertion may be subject to later-described conditions (e.g. minimum desired exertion/speed of the individual). In this way, for each segment of the intended route, a recommended exertion can be calculated.

Information on the recommended exertion of the individual may be displayed, for example, via the user interface 33. In other examples, information on the recommended exertion may be provided through another sensory output system, such as a speaker or tactile device (e.g. a vibration unit).

In some embodiments, the recommended exertion unit 36 maybe adapted to determine a current recommended exertion of the individual, based upon a current location of the individual as tracked by the individual tracking apparatus 31. In particular, the recommended exertion unit may be adapted to use the determined location of the individual to establish in which segment of the segmented route the individual is currently in, and select the recommended exertion associated with that segment as the current recommended exertion of the individual.

The current recommended exertion of the individual may be provided to the individual via the user interface 33 or another sensory output system.

In some embodiments, the device 30 also comprises exertion monitoring apparatus 37 adapted to determine a current exertion of the individual. This may be obtained using any previously described method of determining a current exertion.

For example, the exertion monitoring apparatus 34 may comprise a vital sign monitor 37a adapted to monitor a vital sign of the individual, which may be indicative of their level of exertion. For example, the vital sign monitor may comprise a microphone or a (chest-worn) motion sensor used to track a breathing of the individual. In other examples, the exertion monitoring apparatus comprises a heart-rate monitor or other vital sign monitor (also indicative of a current exertion).

In another example, the exertion monitoring apparatus 34 may comprise a speed detector 37b adapted to determine a current speed of the individual, such as a speedometer or an accelerometer. A speed of the individual may represent their level of exertion. Of course, it will be appreciated that the method of transportation will affect the exertion provided by an individual for a given speed.

In such embodiments, the recommended exertion calculation unit 36 maybe adapted to receive a current exertion and determine an adjustment to be made to the current exertion to meet a recommended exertion of the individual. By way of example, a current exertion and the recommended exertion may be processed to determine how the current exertion should be adjusted (e.g. increased or decreased) to align with the recommended exertion. Thus, an adjustment to the current exertion can be calculated.

The adjustment to the current exertion may be provided to the individual, for example, via the user interface 33 or any other sensory output system. Of course, an indicator of the current exertion of the individual may also be provided to the individual (e.g. via the user interface 33 or other sensory output system).

It has previously been explained how other factors or variables may be considered when determining a recommended exertion for the individual, i.e. provide conditions to the recommended exertion. Optional modules of the device 30 are hereafter described that take account of possible other variables.

In one such example, the device 30 comprises an individual goal database 38 adapted to store travel criteria desired by the individual. In particular, the individual goal database may store one or more goals or desired outcomes of the individual when travelling along the intended route. The individual goal database 38 may be stored in the memory system 38.

The individual goal database 38 may obtain these goals from the user interface 33, as illustrated, or from an external source such as a server (e.g. providing an exercise guideline). Thus, the user interface 33 may act as an individual goal obtaining unit adapted to obtain one or more goals of the individual when travelling through areas of the route.

The recommended exertion calculation unit 36 may use the one or more goals of the individual when calculating a recommended exertion for each segment of the segmented travel route.

For example, a goal of the individual maybe to complete the intended route (i.e. the journey) within at least a preset time. The recommended exertion calculation unit may be adapted to determine a level of exertion for each segment that enables the individual to meet the preset time whilst also minimizing inhalation of pollution when travelling.

In another example, a goal of the individual may be to include a short period of high-intensity exertion whilst travelling the intended route. The recommended exertion calculation unit may be adapted to determine in which segment(s) this period of exertion can be performed to minimize inhalation of pollutants.

In this way, it will be clear that various desired outcomes of the individual may be taken into account when calculating the recommended exertion(s) for the individual travelling along the intended route.

In some embodiments, the recommended exertion calculation unit may be adapted to take into account a time taken for a change in an individual's exertion level to result in a change in the individual's breathing ("lag time"). It has been recognized that there may be a lag or delay between an individual increasing their level of exertion and a corresponding increase in the breathing rate/depth of the individual and vice versa, due at least to an oxygen debt. Methods of determining an individual's response to changed intensity of exercise are fairly well known in the art.

Thus, the recommended calculation unit may adjust or determine the recommended exertion further based on a lag time for the individual.

For example, consider a scenario in which a segment of the intended route is associated with a very high level of pollution but is very short in length. In such a scenario, the recommended exertion unit may recommend the individual to provide a very high level of exertion to pass through the highly polluted segment to reduce the total amount of pollution inhaled by the individual - as the individual's breathing will not have time to catch up with the increase in exertion before the individual has left the highly polluted segment.

It is herein understood that as an individual moves from a high intensity exertion to a low intensity exertion, there is a delay before a breathing rate/depth decreases, and vice versa. Thus, at transitions between segments of the segmented route, it would be preferable to account for the lag time before the change in segments.

The recommended calculation unit may therefore be adapted to account for a lag time at transitions between segments.

For example, when moving from a lowly polluted area (where the individual can provide high exertion levels) to a highly polluted area (where the individual may only be able to provide a low exertion level), the recommended calculation unit may be adapted to encourage the individual to provide a low exertion toward the end of the lowly polluted area - so that the breathing rate/depth of the individual can catch up with the change to exertion before entering the highly polluted area.

By way of another example, as an individual is approaching the end of a highly polluted area, they may be encouraged to speed up (i.e. provide greater exertion) so that they more quickly leave the highly polluted area without breathing in additional pollutants as a result from heavy/fast breathing - as the individual's breathing rate/depth will not have the opportunity to catch up with the increased exertion before exiting the highly polluted area.

The lag time of the individual may be obtained, for example, from the characteristic data stored in the individual characteristic database 34. In other examples, a lag time may be a predetermined value (e.g. a universal average of lag times). In yet other examples, the lag time may be calculated based on characteristics of the individual (e.g. age, gender, fitness and so on).

In some embodiments, the device 20 further comprises enjoyment determining apparatus 39a, adapted to predict an enjoyment of the individual at different portions of the intended route.

The predicted enjoyment may, for example, be based on characteristics of the route, e.g. stored in the mapping database 32. For example, a high enjoyment may be predicted if it is known that a portion of the route has a good view, and a low enjoyment may be predicted if it is known that a portion of the route is associated with bad smells. The predicted enjoyment may, for example, be made on a scale of 0-10, as a percentage, or as a discrete prediction (e.g. HIGH, MEDIUM or LOW).

The recommended calculation unit 36 may calculate an exertion of the individual further based on the predicted enjoyment of the portions of the route.

By way of example, a recommended exertion may be reduced for a portion of the route associated with a high enjoyment (so that the individual has more time to enjoy the portion). A recommended exertion may be increased for a portion of the route associated with a low enjoyment (so that the individual can quickly move past the unenjoyable part of the route).

In particular, the recommended calculation unit may be adapted to maximize an enjoyment of the individual, whilst satisfying other conditions for the exertion of the individual (e.g. a maximum allowable exertion set based on a pollution level and optionally the individual's capabilities, or a minimum allowable exertion based on an individual's goals).

This allows an individual's exposure to pollution to be reduced, monitored or otherwise taken account of whilst also taking account of the individual's enjoyment.

In at least one example, the device 30 is adapted to receive individual feedback (e.g. via the user interface 33) about their enjoyment of the journey (e.g. by indicating enjoyable sections or parts of the route). This may be stored (e.g. in the mapping database 32) and used as a basis for calculating an enjoyment of future journeys using the device 30. Other methods/devices for obtaining feedback about an enjoyment would be readily recognized by the skilled person, such as sensors. Such sensors may include cameras (e.g. for capturing facial expressions indicative of enjoyment level), galvanic skin sensors (for measuring stress/relaxation indicators) or heart rate variability monitors (for measuring stress/relaxation heartbeat indicators).

In some embodiments, the recommended exertion calculation unit 36 is adapted to calculate a recommended exertion further based on characteristics of the terrain, in particular potential forced changes to an exertion level of the individual due to hazards or obstacles along the intended route, such as traffic lights, heavy traffic, road crossings etc.

Such forced changes to an exertion level of an individual may typically be associated with changes in a pollution level around the individual. It would therefore be advantageous to take account of such forced changes.

Moreover, such forced changes will also affect a speed of the individual, meaning that a predicted time that an individual will spend within a particular segment of the route will increase, which could affect a calculation of a recommended exertion (for the remainder of that segment).

By way of example, the recommended exertion calculation unit may recommend that an exertion level be decreased upon approaching a road crossing, so that an individual is not breathing heavily/deeply when waiting (e.g. to be allowed to cross) in the vicinity of a high pollution level environment, thereby reducing their exposure to pollution.

By way of another example, the recommended exertion calculation unit may recommend that an individual provide a lower level of exertion throughout a segment, as it is known that they will be required to wait for an obstacle to clear, thereby increasing their time spent in the segment.

Other examples for modifying a recommended exertion based on terrain characteristics will be readily apparent to the skilled person. Information on characteristics of the terrain, e.g. potential forced changes, including location and/or severity, may be stored, in the mapping database 32.

In some embodiments, the mapping data may also be temporally resolved. That is, the mapping data may include predicted future pollution levels for locations along the individual's intended route. The predicted future pollution level may be used to more accurately determine a predicted inhalation of pollutants by the individual and/or to more accurately recommend a level of exertion that takes account of the expected pollution level along the route (i.e. which may change over time).

In preferable examples, the recommended exertion calculation unit may be able to give a suggested time for performing the journey, e.g. after pollution levels have changed or decreased, in order to meet individuals goals of the journey (such as a desire to maintain a minimum exertion level). By way of example, the recommended exertion calculation unit may be adapted to give a notification when a level of pollution has decreased so that the individual may travel at speed along the entirety of the intended route without

Thus, the recommended exertion calculation unit may be adapted to further take into account a change of pollution levels over time.

In some examples, the characteristic data stored by the individual characteristic database 34 may include data on the individual's sensitivity to different pollutants or pollution types (e.g. pollen or exhaust fumes). The recommended exertion calculation unit 36 may be adapted to alter a recommended level of exertion based on the sensitivity of the individual to different pollutant types.

By way of example, the recommended exertion calculation unit may prioritize a slow breathing rate in an area of high pollen count. This function may also be used to coach the individual on management of their allergy as part of a wider allergy management system.

In some embodiments, the device 30 comprises a route determiner 39b, which is adapted to identify alternative routes that may avoid areas with high pollution. The route determiner 39b may, for example, use a mapping software to identify alternative routes for the individual and use the recommended exertion calculation unit to calculate recommended exertion levels along each route.

Thus, the recommended exertion calculation unit may determine a recommended exertion level along a plurality of different routes, and the route determiner 39b may be adapted to identify suitable routes for the individual that minimizes their exposure to pollution.

In other words, the afore-mentioned modules/units may be used to calculate multiple possible routes and associated recommend exertion level(s) for the individual, and the recommended exertion calculation unit may be adapted to identify a route based on an amount of pollution inhaled by the individual.

As previously described, in possible embodiments the recommended exertion calculation unit may be adapted to calculate a recommended exertion (for each segment) further based on characteristics of the individual (e.g. stored by the individual characteristic database 34).

By way of example, a fitness level (e.g. likelihood an individual will be able to go at a particular speed) may provide an upper limit on possible recommended exertions for the individual.

A recommended exertion may be based on a type of transportation used by the individual. For example, where a recommended exertion is a speed, a recommended exertion will be higher if the individual is travelling by bicycle then if they are travelling by foot.

Whilst the above described embodiments has been described in the context of calculating a recommended for each segment of an intended route, it will be appreciated that aspects may be applied to other embodiments, for example, when performing a reactionary calculation (e.g. as described with reference to Figure 2) and so on. It is therefore not essential for the recommended exertion calculation unit to know an intended route of the individual to employ some the above-described variants of the embodiment.

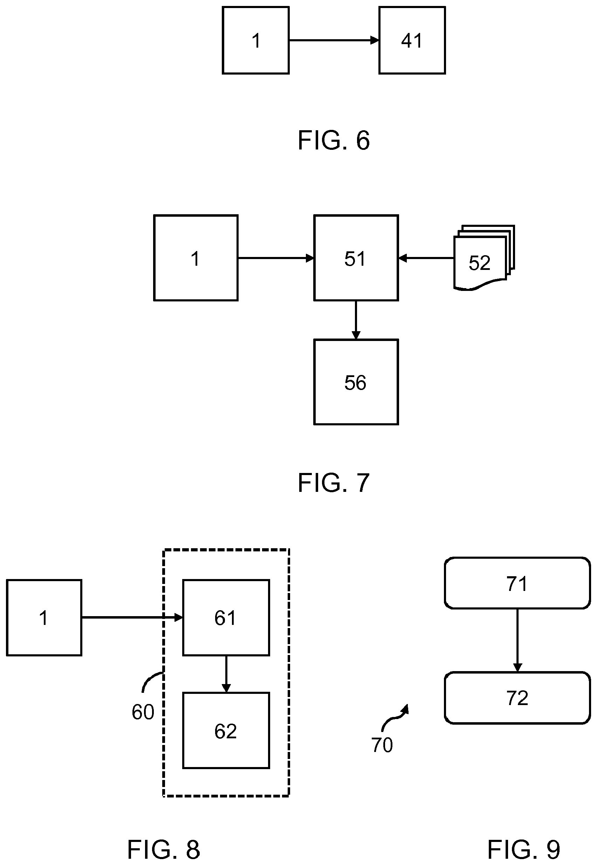

Figures 6 and 7 illustrate different embodiments for notifying an individual about their recommended level of exertion.

In Figure 6, the system 1 is adapted to generate display data indicating the recommended level of exertion or change to exertion for the individual. A display 41 receives the display data and displays it (e.g. via a screen). This provides a visual indication of the recommended level of exertion or recommended change to exertion.

In Figure 7, the system 1 is adapted to generate a recommended level of exertion, such as a recommended speed or recommended pace for the individual.

A song selector 51 selects a playlist of songs, from a database 52 of songs, based on the recommended level of exertion. Each song is matched to a recommended exertion of the individual, e.g. based on the tempo or speed of the song(s).

It has been recognized that individuals naturally synchronize their movements to listened music when walking/jogging. By selecting songs based on a recommended level of exertion, the songs can be used to modulate or control the tempo of an individual's movement, thereby increasing a likelihood that the individual complies with the recommended exertion. In this way, a playlist of songs can be selected that matches the recommended exertion of the individual.

In preferable embodiments, where a plurality of different recommended exertions are generated (e.g. for different points along a route), the length of the songs may be adapted so that it matches the different levels and timings of exertion.

An output device 54 or output connection (such as a 3mm jack) outputs the selected songs to the individual, e.g. via a speaker.

Other methods of notifying an individual as to their recommended exertion will be apparent to the skilled person, e.g. using a haptic output mechanism such as a vibration element.

Figure 8 illustrates another embodiment for utilizing the recommended exertion of the individual output by the system 1. The embodiment of Figure 8 may be used where the individual is travelling using an electrically assisted, but manually powered transportation device, such as an electric bicycle ("e-bike").

There is provided an individual assistance device 60 adapted to reduce an exertion of the individual exertion when travelling. The individual assistance device 60 comprises an assistance control unit 61 and an assistance provider 62.

The assistance control unit 61 is adapted to control the operation of the assistance provider 62 based on the recommended exertion of the individual.

The assistance provider 62 may be a motor of an electric bicycle. As would be understood by the skilled person, such an assistance provider is adapted to vary a level of assistance, e.g. electrical power applied by the motor, in order to change/control a speed of the electrical device to thereby assist the individual using the electric bicycle.

The assistance control unit 60 may be adapted to maintain a predetermined speed of the transportation device. Thus, in response to a recommended exertion dropping, the assistance control unit 61 may increase the assistance provided by the assistance provider 62.

Where recommended exertions are known over the course of an intended journey, the assistance control unit 61 may be adapted to generate a plan for the assistance provider 62 based on the recommended exertions. This plan may aim to maintain a minimum speed of the individual, or ensure they arrive at a destination before a predetermined time, whilst ensuring that their inhalation of pollution remains below a desired or recommended level.

Thus, the individual assistance device is adapted to vary a level of assistance based on the determined pollution level of the area through which the individual is to travel.

From the foregoing description, it will be clear that there is provided a system having a recommended exertion calculation unit that recommend a level or levels of exertion. The recommended level of exertion may be calculated to minimize pollution inhalation, whilst also satisfying a number of other conditions.

The conditions are determined based on a number of variables. In particular, different variables can be used to establish a range of allowable levels of exertion for the individual, which can be used to establish the recommended level of exertion. Possible variables include an: individual's enjoyment of the journey, individual's capabilities and/or characteristics and the individual's desired goals or targets.

For example, an individual's capabilities may establish a maximum allowable level of exertion for the individual (e.g., what the individual is capable of doing). An individual's goals may establish a minimum level of exertion (i.e. a minimum level of exertion that the individual wants).

Preferably, the recommended exertion calculation unit is adapted to select a recommended level(s) of exertion that meets or is a compromise of these conditions whilst also minimizing a level of pollution inhaled by the individual.

Any herein described system may further comprise a fitness tracking module, adapted to determine a fitness level or fitness data of the individual. The fitness tacking module may be adapted to analyze the exertion of the individual over time, together with the routes (e.g. length and/or change in incline) taken by the individual and/or speed of the individual, to determine their fitness level. This may be performed, for example, by performing an active monitoring of the exertion of the individual compared to a predicted exertion (e.g. based on standard values) for the location.

Figure 9 illustrates an embodiment for a method 70 for determining a recommended exertion rate of an individual when travelling through an area.

The method comprises a step 71 of determining a pollution level of the area through which the individual is to travel; and a step 72 of calculating, based on the determined pollution level, a recommended exertion of the individual that minimizes the individual's inhalation of pollution when travelling through the area.

The method 70 may be appropriately adapted to comprise steps performed by any aspect of the previously described system for determining a pollution level.

As discussed above, embodiments make use of a system. The system can be implemented in numerous ways, with software and/or hardware, to perform the various functions required. A processor is one example of a system that employs one or more microprocessors that maybe programmed using software (e.g., microcode) to perform the required functions. A system may however be implemented with or without employing a processor, and also maybe implemented as a combination of dedicated hardware to perform some functions and a processor (e.g., one or more programmed microprocessors and associated circuitry) to perform other functions.

Examples of system components that may be employed in various embodiments of the present disclosure include, but are not limited to, conventional microprocessors, application specific integrated circuits (ASICs), and field-programmable gate arrays (FPGAs).

In various implementations, a processor or system may be associated with one or more storage media such as volatile and non-volatile computer memory such as RAM, PROM, EPROM, and EEPROM. The storage media may be encoded with one or more programs that, when executed on one or more processors and/or systems, perform the required functions. Various storage media may be fixed within a processor or system or may be transportable, such that the one or more programs stored thereon can be loaded into a processor or system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system (1) for determining a recommended exertion rate of an individual when travelling through an area, the system comprising:
pollution sensing apparatus (2) adapted to determine a pollution level of the area through which the individual is to travel; and
a recommended exertion calculation unit (3) adapted to calculate, based on the determined pollution level, a recommended exertion of the individual that takes into account the individual's resulting inhalation of pollution when travelling through the area.

2. The system of claim 1, wherein the recommended exertion calculation unit is further adapted to determine, using the determined pollution level, a relationship between an amount of pollution inhaled by the individual and an exertion of the individual,
wherein recommended exertion calculation unit is adapted to calculate the recommended exertion of the individual based on the relationship between an amount of pollution inhaled by the individual and an exertion of the individual.

3. The system of claim 1 or 2, further comprising exertion monitoring apparatus (3) adapted to determine a current exertion of the individual,
wherein the recommended exertion calculation unit is adapted to calculate a recommended adjustment to the individual's exertion based on the recommended exertion and the current exertion of the individual.

4. The system of claim 3, wherein the exertion monitoring apparatus comprises a vital sign monitoring system adapted to monitor a vital sign of the individual.

5. The system of claim 4, wherein the exertion monitoring apparatus comprises a respiratory rate monitor adapted to determine a current respiratory rate of the individual, representative of the current exertion of the individual.

6. The system of any of claims 1 to 5, further comprising a sensory output system (41, 56) adapted to provide a sensory output indicative of the recommended exertion of the individual.

7. The system of any of claims 1 to 6, further comprising an individual characteristic determining unit (5) adapted to determine one or more characteristics of the individual,
wherein the recommended exertion calculation unit is adapted to calculate the recommended exertion of the individual further based on the determined one or more characteristics of the individual.

8. The system of any of claims 1 to 7, further comprising terrain determining apparatus (6) adapted to determine one or more characteristics of the terrain in the area through which the individual is to travel,
wherein the recommended exertion calculation unit is adapted to calculate the recommended exertion of the individual further based on the determined one or more characteristics of the terrain.

9. The system of any of claims 1 to 8, further comprising a route determining unit (7) adapted to determine an intended route of the individual,
wherein the recommended exertion calculation unit is adapted to calculate the recommended exertion of the individual further based on the intended route of the individual.

10. The system of claim 9, wherein the pollution sensing apparatus is adapted to determine pollution levels along the intended route of the individual,
wherein the recommended exertion calculation unit is adapted to calculate the recommended exertion of the individual further based on the determined pollution levels along the intended route of the individual.

11. The system of any of claims 1 to 10, further comprising an individual goal obtaining unit (8) adapted to obtain one or more goals of the individual when travelling through the area,
wherein the recommended exertion calculation unit is adapted to calculate the recommended exertion of the individual further based on the determined one or more goals of the individual.

12. The system of any of claims 1 to 11, further comprising an individual assistance device (60) adapted to reduce an exertion of the individual exertion when travelling,
wherein the individual assistance device is adapted to vary a level of assistance based on the determined pollution level of the area through which the individual is to travel.

13. A method (70) for determining a recommended exertion rate of an individual when travelling through an area, the method comprising:
determining (71) a pollution level of the area through which the individual is to travel; and
calculating (72), based on the determined pollution level, a recommended exertion of the individual that minimizes the individual's inhalation of pollution when travelling through the area.

14. The method of claim 13, further comprising:
determining a current exertion of the individual; and
calculating a recommended adjustment to the individual's exertion based on the recommended exertion and the current exertion of the individual.

15. A computer program comprising code means for implementing the method of any one of claims 13 or 14 when said program is run on a computer.
